(19) Europäisches Patentamt
European Patent Office
Office européen des brevets

(11) Veröffentlichungsnummer : **0 393 667 B1**

# (12) EUROPÄISCHE PATENTSCHRIFT

(45) Veröffentlichungstag der Patentschrift :
**23.06.93 Patentblatt 93/25**

(51) Int. Cl.$^5$ : **C07C 43/164,** C07C 41/28, C07C 43/205, C07C 43/168

(21) Anmeldenummer : **90107435.1**

(22) Anmeldetag : **19.04.90**

## (54) Verfahren zur Herstellung von Benzylmethylethern.

(30) Priorität : **21.04.89 DE 3913163**

(43) Veröffentlichungstag der Anmeldung :
**24.10.90 Patentblatt 90/43**

(45) Bekanntmachung des Hinweises auf die Patenterteilung :
**23.06.93 Patentblatt 93/25**

(84) Benannte Vertragsstaaten :
**CH DE FR GB LI**

(56) Entgegenhaltungen :
**EP-A- 0 059 373**
**EP-A- 0 217 089**
**EP-A- 0 299 286**
**EP-A- 3 804 162**

(73) Patentinhaber : **BASF Aktiengesellschaft**
**Carl-Bosch-Strasse 38**
**W-6700 Ludwigshafen (DE)**

(72) Erfinder : **Steck, Werner, Dr.**
**Auerstrasse 4**
**W-6700 Ludwigshafen (DE)**
Erfinder : **Lermer, Helmut, Dr.**
**Bockenheimer Strasse 12**
**W-6700 Ludwigshafen (DE)**
Erfinder : **Hermeling, Dieter, Dr.**
**Zum Ordenswald 73 f**
**W-6730 Neustadt (DE)**
Erfinder : **Grosse Brinkhaus, Karl-Heinz, Dr.**
**Kaiserslauterer Strasse 112**
**W-6702 Bad Duerkheim (DE)**

**EP 0 393 667 B1**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft ein Verfahren zur Herstellung von Benzylmethylethern durch heterogen katalysierte Umlagerung von Benzaldehyddimethylacetalen an sauren Heterogenkatalysatoren.

Benzylmethylether werden als Riechstoffe und Zusätze zu Kosmetika, Seifen, Bleichmittel, Detergentien aber auch zur Herstellung wärmebeständiger Kunstharze verwendet.

Zu ihrer Herstellung sind bereits eine Reihe von Synthesemethoden beschrieben. So kann Benzylmethylether beispielsweise durch Umsetzung von Benzylalkohol mit Methanol in Gegenwart eines sauren Homogenkatalysators gewonnen werden. Benzylmethylether können auch durch Reaktion von Natriummethylat mit Benzylchlorid oder mit Benzylalkohol erhalten werden (Kirk-Othmer, Encyclopedia of Chemical Technology; Vol. 5, 1979, Seite 829 bzw. Vol. 3, Seite 794). Die Alkylierung von Benzylalkohol mit Dimethylsulfat bzw. die Umsetzung von Diazomethan mit Benzylalkohol in Gegenwart von $AlCl_3$ werden bei Houben-Weyl, Methoden der organischen Chemie im Band VI/1a, Seite 33 (1965) bzw. im Band X/4, Seite 659 (1968) beschrieben. Im Band IV/1d, Seite 283, wird die Herstellung von Benzylmethylethern durch Reduktion von Aldehyden mit Triethylsilan in Gegenwart von Methanol und Schwefelsäure behandelt. Auf Seite 431 des Bandes IV/1d wird Benzylmethylether aus Benzaldehyddimethylacetal durch Reduktion mit Lithiumalanat in Gegenwart von Aluminiumchlorid und Schwefelsäure beschrieben. Schließlich lassen sich Benzylmethylether durch Reduktion von Ketonen mit Triethylsilan in Gegenwart von Schwefelsäure synthetisieren (Band XIII/5, Seite 352 (1980).

Die aufgeführten Methoden werden entweder homogenkatalytisch durchgeführt, was zu Problemen bei der Abtrennung des Katalysators führt oder sie verwenden schwierig handhabbare Reagentien.

Der vorliegenden Erfindung lag daher die Aufgabe zugrunde, den zuvor genannten Nachteilen abzuhelfen und einen neuen und besseren Zugang zu Benzylmethylethern zu finden.

Demgemäß wurde ein neues und verbessertes Verfahren zur Herstellung von Benzylmethylethern I gefunden, welches dadurch gekennzeichnet ist, daß man Benzaldehyddimethylacetale II in der Gasphase an sauren Heterogenkatalysatoren umsetzt.

Die Benzylmethylether I sind nach folgender Methode erhältlich:

Die Umsetzung erfolgt durch Kontakt eines Benzaldehyddimethylacetals II mit einem Heterogenkatalysator nach folgender Reaktionsgleichung:

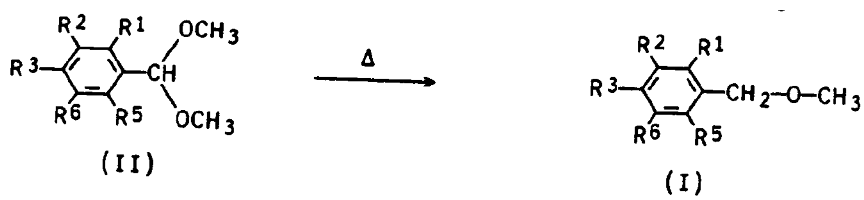

Der der Erfindung zugrundeliegende Reaktionsmechanismus ist unbekannt, entspricht jedoch formal einer Formaldehydabspaltung aus dem Dimethylacetal. In einigen Umsetzungen konnte im Abgas der Reaktion Formaldehyd sowohl qualitativ durch Bildung eines metallischen Silberspiegels aus ammoniakalischer Silbernitratlösung als auch gaschromtatographisch nachgewiesen werden.

Die Reaktion kann diskontinuierlich oder vorzugsweise kontinuierlich in der Gasphase bei 50 bis 500°C und 0,01 bis 50 bar durchgeführt werden.

Die Gasphasenreaktion kann beispielsweise bei Temperaturen von 100 bis 500°C, vorzugsweise bei 150 bis 400°C und Drucken von 0,1 bis 50 bar, besonders bevorzugt bei 200 bis 300°C und Drucken von 0,5 bis 5 bar durchgeführt werden. Bei der Umsetzung in der Gasphase hält man vorteilhaft eine Katalysatorbelastung von 0,1 bis 20, insbesondere von 0,5 bis 10 g Ausgangsstoff der Formel II je g Katalysator und Stunde ein (WHSV = g Einsatzgemisch/g Katalysator und Stunde). Die Gasphasenreaktion kann in einem Festbett, in einem Fließbett oder in einem Wirbelbett ausgeführt werden.

Dabei empfiehlt es sich je nach Reaktionstemperatur und Thermostabilität der beteiligten Moleküle gegebenfalls die Kontaktzeit der Reaktionspartner am Katalysator zu verkürzen und/oder das Reaktionsgemisch nach dem Durchlaufen der Katalysatorschüttung rasch abzukühlen. Auf diese Weise lassen sich auch bei thermolabilen Einsatzstoffen und/oder Endprodukten ausbeutemindernde Nebenreaktionen vermeiden oder zumindest stark zurückdrängen. Auch durch eine Vakuumfahrweise, z. B. mittels einer dem Katalysatorbett austragseitig nachgeschalteten Vakuumpumpe, können aufgrund der auf diese Weise realisierbaren kurzen, auch im Bruchteil von Sekunden liegenden Verweilzeiten am Katalysator die Selektivität und Ausbeuten vorteilhaft beeinflußt werden. Die Durchführung erfolgt bei allen Varianten vorzugsweise kontinuierlich, wobei auch eine Kreisgasfahrweise mit vollständiger oder teilweiser Rückführung des Reaktionsaustrages angebracht sein kann.

Nach der Umsetzung werden die entstandenen Produkte durch übliche Verfahren, z.B. durch Destillation aus dem Reaktionsgemisch isoliert; nichtumgesetzte Ausgangsstoffe werden gegebenenfalls in die Umsetzung zurückgeführt.

Die Substituenten $R^1$ bis $R^5$ in Formel I und II haben vorzugsweise folgende Bedeutung:

$R^1$ bis $R^5$ unabhängig voneinander

- Wasserstoff,
- Hydroxy,
- Halogen wie Fluor, Chlor, Brom und Iod, bevorzugt Fluor, Chlor und Brom, besonders bevorzugt Fluor und Chlor
- Alkyl, vorzugsweise unverzweigtes oder verzweigtes $C_1$-bis $C_{12}$-Alkyl, wie Methyl, Ethyl, n-Propyl, iso-Propyl, n-Butyl, iso-Butyl, sec.-Butyl, tert.-Butyl, n-Pentyl, iso-Pentyl, sec.-Pentyl, tert.-Pentyl, neo-Pentyl, 1,2-Dimethylpropyl, n-Hexyl, iso-Hexyl, sec.-Hexyl, n-Heptyl, iso-Heptyl, n-Octyl, iso-Octyl, n-Nonyl, iso-Nonyl, n-Decyl, iso-Decyl, n-Undecyl, iso-Undecyl, n-Dodecyl und iso-Dodecyl,
- Cycloalkyl, vorzugsweise $C_3$- bis $C_8$-Cycloalkyl, wie Cyclopropyl, Cyclobutyl, Cyclopentyl, Cyclohexyl, Cycloheptyl und Cyclooctyl,
- Aryl, bevorzugt Phenyl, 1-Naphthyl und 2-Naphthyl, besonders bevorzugt Phenyl,
- Alkylaryl, vorzugsweise ein- bis dreifach durch $C_1$- bis $C_4$-Alkyl substituiertes Aryl, wie 2-Methylphenyl, 3-Methylphenyl, 4-Methylphenyl, 2-Ethylphenyl, 3-Ethylphenyl, 4-Ethylphenyl, 2-n-Propylphenyl, 3-n-Propylphenyl, 4-n-Propylphenyl, 3-iso-Propylphenyl, 4-iso-Propylphenyl, 2-n-Butylphenyl, 3-n-Butylphenyl, 4-n-Butylphenyl, 3-iso-Butylphenyl, 4-iso-Butylphenyl, 3-sec.-Butylphenyl, 4-sec.-Butylphenyl, 3-tert.-Butylphenyl, 4-tert.-Butylphenyl, 2,4-Dimethylphenyl, 2,6-Dimethylphenyl, 3,5-Dimethylphenyl und 2, 4, 6-Trimethylphenyl,
- Alkoxy, vorzugsweise unverzweigtes oder verzweigtes $C_1$-bis $C_{12}$-Alkoxy wie Methoxy, Ethoxy, n-Propoxy, iso-Propoxy, n-Butoxy, iso-Butoxy, sec.-Butoxy, tert.-Butoxy, n-Pentoxy, iso-Pentoxy, sec.-Pentoxy, tert.-Pentoxy, neo-Pentoxy, 1,2-Dimethylpropoxy, n-Hexoxy, iso-Hexoxy, sec.-Hexoxy, n-Heptoxy, iso-Heptoxy, n-Octoxy, iso-Octoxy, n-Nonoxy, iso-Nonoxy, n-Decoxy, iso-Decoxy, n-Undecoxy, iso-Undecoxy, n-Dodecoxy und iso-Dodecoxy,

Besonders bevorzugte Verbindungen der Formeln I und II sind solche, bei denen drei oder vier der Reste $R^1$ bis $R^5$ für Wasserstoff stehen.

Als Ausgangsstoffe geeignete Dimethylacetale II sind beispielsweise:
2-Methoxybenzaldehyddimethylacetal,
4-Methoxybenzaldehyddimethylacetal,
2-Ethoxybenzaldehyddimethylacetal,
4-Ethoxybenzaldehyddimethylacetal,
tert-Butoxyverbindungen, wie 4-t-Butoxybenzaldehyddimethylacetal
4-Benzyloxybenzaldehyddimethylacetal,
2-Methylbenzaldehyddimethylacetal,
4-Methyl-benzaldehyddimethylacetal,
4-Isopropyl-benzaldehyddimethylacetal,
4-tert-Butyl-benzaldehyddimethylacetal,
3-tert-Butyl-4-methoxybenzaldehyddimethylacetal,
4-(1-Methoxy-1-methylethyl)benzaldehyddimethylacetal,
4-Fluorbenzaldehyddimethylacetal,
3-Cyclopentyl-2-chlorbenzaldehyddimethylacetal,
4-Fluor-2-chlorbenzaldehyddimethylacetal.

Die Ausgangsstoffe sind bekannt oder nach allgemein bekannten Methoden durch elektrochemische Oxidation in Gegenwart von Methanol (EP-A-129 795 und DE-A-28 48 397) herstellbar.

Als Katalysatoren für das erfindungsgemäße Verfahren werden zeolithische Katalysatoren eingesetzt. Zeolithe sind kristalline Aluminosilikate, die eine hochgeordnete Struktur mit einem dreidimensionalen Netzwerk von $SiO_4$- oder $AlO_4$-Tetraedern besitzen, die durch gemeinsame Sauerstoffatome verbunden sind. Das Verhältnis der Si- und Al-Atome zu Sauerstoff beträgt 1:2. Die Elektrovalenz der Aluminium enthaltenden Tetraeder ist durch Einschluß von Kationen in den Kristall, z.B. eines Alkali- oder Wasserstoff-ions ausgeglichen. Ein Kationenaustausch ist möglich. Die Räume zwischen den Tetraedern sind vor der Dehydration durch Trocknen bzw. Calcinieren von Wassermolekeln besetzt.

In den Zeolithen können anstelle von Aluminium auch andere Elemente wie B, Ga, Fe, Cr, V, As, Sb oder Be in das Gitter eingebaut werden, oder das Silicium kann durch ein vierwertiges Element wie Ge, Ti, Zr, Hf ersetzt werden.

Entsprechend ihrer Struktur werden die Zeolithe in verschiedene Gruppen unterteilt. So bilden bei der Mordenit-Gruppe Ketten oder bei der Chabasit-Gruppe Schichten aus Tetraedern die Zeolith-Struktur, während sich bei der Faujasit-Gruppe die Tetraeder zu Polyedern ordnen, z. B. in Form eines Kubooktaeders, der aus Vierringen bzw. Sechsringen aufgebaut ist. Je nach Verknüpfung der Kubooktaeder, wodurch unterschiedlich große Hohlräume und Poren entstehen, unterscheidet man Zeolithe vom Typ A, L, X oder Y. Die Zeolithe X und Y sind Zeolithe des Faujasit-Typs.

Für das erfindungsgemäße Verfahren geeignet sind auch Zeolithe vom Pentasiltyp. Diese haben als Grundbaustein einen aus $SiO_4$-Tetraedern aufgebauten Fünfring gemeinsam. Sie sind durch ein hohes $SiO_2/Al_2O_3$-Molverhältnis - auch Modul genannt - gekennzeichnet, sowie durch Porengrößen, die zwischen denen der Zeolithe vom Typ A und denen vom Typ X oder Y liegen. Für das erfindungsgemäße Verfahren haben sich beispielsweise Pentasil-Zeolithe mit $SiO_2/Al_2O_3$-Verhältnissen zwischen 20 und 700 als gut geeignet erwiesen. Die Herstellung dieser Pentasil-Zeolithe wird u.a. in den US-A-3 702 886 und US-A-3 709 979 beschrieben. Die Herstellung des analogen Borosilikatzeolithen AMS-1B ist Gegenstand der DE-A-27 46 790.

Die erfindungsgemäß verwendbaren Zeolithe können unterschiedliche chemische Zusammensetzung aufweisen, geeignet sind z. B. Alumino-, Boro-, Eisen-, Beryllium-, Gallium-, Chrom-, Arsen-, Antimon-, Titan- und Wismutsilikatzeolithe oder deren Gemische sowie Alumino-, Boro-, Gallium-, Eisengermanatzeolithe oder deren Gemische. Insbesondere eignen sich die Alumino-, Boro- oder Eisensilikatzeolithe des Pentasiltyps für das erfindungsgemäße Verfahren. Der Aluminosilikatzeolith wird z. B. aus einer Aluminiumverbindung, vorzugsweise $Al(OH)_3$ oder $Al_2(SO_4)_3$ und einer Siliciumkomponente, wie hochdisperses Siliciumdioxid, Kieselgele oder Kieselsole in wäßriger Aminlösung, insbesondere in Polyaminen wie 1,6-Diaminohexan oder 1,3-Diaminopropan oder Triethylentetramin-Lösung mit oder insbesondere ohne Alkali- oder Erdalkalizusatz bei 100 bis 220°C unter autogenem Druck hergestellt. Auch gehören hierzu die isotaktischen Zeolithe nach EP-A-34 727. Die erhaltenen Aluminosilikatzeolithe enthalten je nach Wahl der Einsatzstoffmengen ein $SiO_2/Al_2O_3$-Verhältnis von 10 bis 40 000.

Auch lassen sich derartige Aluminosilikatzeolithe in etherischem Medium wie Diethylenglykoldimethylether, in alkoholischem Medium wie Methanol oder 1,4-Butandiol oder in Wasser synthetisieren.

Der Borosilikatzeolith wird z. B. bei 90 bis 200°C unter autogenem Druck synthetisiert, indem man eine Borverbindung, z.B. $H_3BO_3$, mit einer Siliciumverbindung, wie hochdispersem Siliciumdioxid in wäßriger Aminlösung, insbesondere in 1,6-Diaminohexan oder 1,3-Diaminopropan oder Triethylentetramin-Lösung mit und insbesondere ohne Alkali- oder Erdalkalizusatz zur Reaktion bringt. Auch isotaktische Borosilikatzeolithe, hergestellt nach EP-A-34 727 kann man vorteilhaft verwenden. Man kann auch Borosilikatzeolithe verwenden, die statt aus wäßriger Aminlösung aus einer etherischen Lösung, z.B. Diethylenglykoldimethylether oder aus alkoholischer Lösung, z. B. 1,6-Hexandiol auskristallisiert werden. Die Herstellung von Borzeolith wird z. B. in EP-A-7 081 beschrieben. Für das erfindungsgemäße Verfahren haben sich beispielsweise Borzeolithe mit einem $SiO_2/B_2O_3$-Verhältnis zwischen 30 und 800 als gut geeignet erwiesen.

Den Eisensilikatzeolith erhält man z.B. aus einer Eisenverbindung, vorzugsweise $Fe_2(SO_4)_3$ und einer Siliciumverbindung, wie hochdispersem Siliciumdioxid in wäßriger Aminlösung, insbesondere 1,6-Diaminohexan mit oder ohne Alkali-oder Erdalkalizusatz bei 100 bis 200°C unter autogenem Druck. Die Herstellung wird beschrieben in EP-A-10 572.

Zu den verwendbaren siliciumreichen Zeolithen ($SiO_2/Al_2O_3$ größer oder gleich 10) gehören auch die bekannten ZSM-Typen sowie Ferrierit (EP-A-12 473) und NU-1 (US-A-4 060 590) sowie Silicalite® (US-PS 4 061 724). Auch Aluminosilikatzeolithe, welche neben Aluminium gleichzeitig mindestens ein weiteres dreiwertiges Kation wie Bor, Eisen oder Gallium enthalten, sind geeignet.

Die pulverförmig anfallenden Zeolithe können nach ihrer Synthese, Isolierung, Trocknung bei 100 bis 160°C und Calcinierung bei 450 bis 550°C, mit einem Bindemittel im Massenverhältnis Zeolith zu Bindemittel 90:10 bis 40:60 Gew.% zu Formkörpern wie Strängen oder Tabletten verformt werden. Als Bindemittel eignen sich diverse Aluminiumoxide, bevorzugt Boehmit, amorphe Aluminosilikate mit einem $SiO_2/Al_2O_3$-Verhältnis von 25:75 bis 90:5, $SiO_2$, $TiO_2$, $ZrO_2$, Gemische aus hochdispersem $SiO_2$ und hochdispersem $Al_2O_3$ sowie Ton. Nach der Verformung werden die Extrudate oder Preßlinge bei 110°C/16 h getrocknet und bei Temperaturen um 500°C calciniert.

Man erhält auch gut geeignete Katalysatoren, wenn der isolierte Alumino-bzw. Borosilikatzeolith direkt nach der Trocknung verformt wird und erst nach der Verformung einer Calcinierung unterworfen wird. Die Alumino- und Borosilikatzeolithe können in reiner Form, ohne Binder, als Stränge oder Tabletten eingesetzt werden, wobei als Verstrangungs- oder Peptisierungsmittel z.B. Ethylcellulose, Stearinsäure, Kartoffelstärke, Ameisensäure, Oxalsäure, Essigsäure, Salpetersäure, Ammoniak, Amine, Silikoester und Graphit oder deren Gemische verwendet werden können.

Liegt der Zeolith aufgrund der Art seiner Herstellung nicht in der katalytisch aktiven, aciden H-Form vor, sondern z. B. in der Na-Form, dann kann diese durch Ionenaustausch, z.B. mit Ammoniumionen, und anschließende Calcinierung oder durch Behandlung mit Säuren vollkommen oder partiell in die gewünschte H-Form

überführt werden.

Wenn bei der erfindungsgemäßen Verwendung der zeolithischen Katalysatoren eine durch Koksabscheidung bedingte Desaktivierung eintritt, empfiehlt es sich, die Zeolithe durch Abbrennen der Koksablaqerung mit Luft oder mit einem Luft/$N_2$-Gemisch bei 400 bis 550°C, bevorzugt 500°C, zu regenerieren. Die Zeolithe erhalten dadurch in der Regel ihre Anfangsaktivität zurück.

Um eine möglichst hohe Selektivität, hohen Umsatz sowie lange Standzeiten zu erreichen, ist es häufig vorteilhaft, die Zeolithe zu modifizieren, wobei der Gehalt an den Modifizierungselementen so zu bemessen ist, daß eine ausreichende Aktivität der Katalysatoren gewährleistet ist.

Eine geeignete Modifizierung der Katalysatoren besteht z. B. darin, daß man den unverformten oder verformten Zeolithen mit Metallsalzen durch einen Ionenaustausch oder durch Imprägnierung dotiert. Als Metalle werden Alkalimetalle, wie Li, K, Cs, Erdalkalimetalle, wie Mg, Ca, Ba; Erdmetalle, wie B, Al, Ga und Übergangsmetalle, wie Cu, Zn, Cr, Mn, Fe, Co, Ni, W und Mo aber auch Edelmetalle wie Pd eingesetzt.

Zweckmäßigerweise führt man die Dotierung so durch, daß man z. B. den verformten Zeolithen in einem Steigrohr vorlegt und bei 20 bis 100°C, z.B. eine wäßrige oder ammoniakalische Lösung eines Halogenids oder eines Nitrats der voranbeschriebenen Metalle überleitet. Ein derartiger Ionenaustausch kann z. B. an der Wasserstoff-, Ammonium-, Alkaliform des Zeolithen vorgenommen werden. Eine weitere Möglichkeit der Metallaufbringung auf den Zeolithen ist gegeben, indem man das zeolithische Material z. B. mit einem Halogenid, einem Nitrat, einem Carbonat oder einem Oxid der voranbeschriebenen Metalle in wäßriger, alkoholischer oder ammoniakalischer Lösung imprägniert. Sowohl an einen Ionenaustausch als auch an eine Imprägnierung schließt sich zumindest eine Trocknung, wahlweise eine abermalige Calcinierung an.

Ein Ionenaustausch des in der H-Form oder Ammonium-Form oder Alkali-Form vorliegenden Zeolithen kann so vorgenommmen werden, daß man den Zeolithen in Strängen oder Pellets in einer Kolonne vorlegt und darüber z.B. eine wäßrige Lösung von Ammoniumchlorid bei leicht erhöhter Temperatur zwischen 30 und 80°C im Kreislauf 15 bis 20 h leitet. Danach wird mit Wasser ausgewaschen, bei ca. 110°C getrocknet und bei ca. 500°C calciniert.

Eine weitere Möglichkeit der Modifizierung besteht darin, daß man das zeolithische Material - vor oder nach ihrer Verformung - einer Behandlung mit Säuren wie Salzsäure, Flußsäure und Phosphorsäure und/oder Wasserdampf unterwirft. Dabei geht man z. B. so vor, daß man Zeolithe in Pulverform mit 1 n Phosphorsäure 1 h bei 80°C behandelt. Nach der Behandlung wird mit Wasser gewaschen, bei 110°C/16 h getrocknet und beispielsweise bei 500°C für 10 Stunden calciniert.

Auch eine Modifizierung mit Phosphor durch Behandlung mit Phosphorsäuren oder ihren Salzen bzw. mit Phosphorsäureestern wie Trimethylphosphat ist möglich.

Weitere Katalysatoren für das erfindungsgemäße Verfahren sind Phosphate, insbesondere Aluminiumphosphate, Siliciumaluminiumphosphate, Siliciumeisenaluminiumphosphate, Cerphosphat, Zirkonphosphate, Borphosphat, Eisenphosphat oder deren Gemische.

Als Aluminiumphosphat-Katalysatoren werden für das erfindungsgemäße Verfahren insbesondere unter hydrothermalen Bedingungen synthetisierte Siliciumaluminophosphate (Akronym SAPO) oder Aluminophosphate (Akronym $AlPO_4$) eingesetzt. Diese kristallinen Festkörper weisen definierte Hohlraum- und Porenstrukturen auf und sind mit den Zeolithen strukturverwandt.

Die Herstellung, Eigenschaften und die Klassifizierung dieser Festkörper auf der Basis von Struktur und chemischer zusammensetzung werden ausführlich von R.M. Barrer, Pure and Appl.Chem. Vol. 58, No. 10, pp. 1317-22, 1986; von E.M. Flanigen et al, Pure and Appl. Chem. Vol. 58, No. 10, pp 1351-58, 1986 oder von N.B. Milestone et al, Stud. Surf. Sci. Catal. 1988, 36 (Methane Convers.), pp 553-62 beschrieben.

Aus den derzeit bekannten Kristallstrukturen und den zahlreichen Elementmodifizierungen sind inzwischen gut 700 verschiedene Kombinationen möglich. Die Bezeichnung der Aluminophosphate erfolgt durch folgende Akronyme $AlPO_4$, SAPO, MeAPO, MeAPSO, ElAPO oer ElAPSO. Dabei bedeutet A oder Al gleich Aluminium, S gleich Silicium, P steht für Phosphor und O für Sauerstoff. Unter Me sind Metallkationen von Fe, Mg, Mn, Co und Zn und unter El Kationen von Be, Ga, Ge, Ti, As, B oder Li zu verstehen. Eine mit Bindestrich angefügte Zahl dient der Kennzeichnung der Kristallstruktur der betreffenden Phase.

Für das erfindungsgemäße Verfahren bevorzugt sind insbesondere die Silicium-haltigen Aluminophosphate (SAPO, MeAPSO oder ELAPSO) wie SAPO-11, SAPO-5, SAPO-20, SAPO-34, SAPO-41 oder SAPO-46.

Synthesen der Aluminophosphate sind u.a. beschrieben für $AlPO_4$ in US-A-4 310 440, für SAPO in US-A-4 440 871, für MeAPO in US-A-4 544 143, EP-A-132 708 und US-A-4 567 029, für MeAPSO in EP-A-158 348, EP-A-158 975 und EP-A-161 491, für ElAPO in US-A-4 500 651 und EP-A-158 976 und für ElAPSO in EP-A-159 624.

Als Phosphatkatalysatoren kann man bei dem Verfahren auch gefällte Aluminiumphosphate einsetzen. Beispielsweise wird ein derartiges Aluminiumphosphat hergestellt, indem 92 g Diammoniumhydrogenphosphat

in 700 ml Wasser gelöst werden. Zu dieser Lösung wird 260 g $Al(NO_3)$ 9 x $H_2O$ in 700 ml Wasser über 2 h zugetropft. Dabei wird der pH-Wert durch gleichzeitige Zugabe von 25 %iger $NH_3$-Lösung bei pH 8 gehalten. Der entstandene Niederschlag wird 12 h nachgerührt, dann abgesaugt und ausgewaschen. Er wird bei 60°C/16 h getrocknet.

Borphosphate für das erfindungsgemäße Verfahren lassen sich beispielsweise durch Mischen und Kneten von konzentrierter Borsäure und Phosphorsäure und durch anschließende Trocknung und Calcination in Inertgas-, Luft- oder Dampfatmosphäre bei 250 bis 650°C, vorzugsweise bei 300 bis 500°C herstellen.

Auf diese Phosphate können durch Imprägnierung (Tränken und Aufsprühen) oder in manchen Fällen auch durch Ionenaustausch Modifizierungskomponenten, wie voran bei den Zeolithen beschrieben, aufgebracht werden. Auch kann wie bei den Zeolithkatalysatoren eine Modifizierung mit Säuren erfolgen.

Geeignete saure Katalysatoren sind auch die sauer wirkenden Oxide von Elementen der III. und IV. Hauptgruppe, insbesondere Oxide wie $Al_2O_3$ oder Siliciumdioxid in Form von Kieselgel, Kieselgur, Quarz oder amorphe Aluminosilicate. Handelsübliches $Al_2O_3$ (D 10-10® oder $SiO_2$ (D 11-10®) sind z.B. gut geeignet. Auch mit Phosphorsäure oder Borsäure getränkte Katalysatoren können eingesetzt werden. Phosphorsäure oder Borsäure wird z.B. auf $SiO_2$- oder $Al_2O_3$-Träger z.B. durch Auftränken oder Versprühen aufgebracht. Ein phosphorsäurehaltiger Katalysator kann beispielsweise durch Auftränken von $H_3PO_4$- oder $NaH_2PO_4$- oder $Na_2HPO_4$-Lösung auf $SiO_2$ und anschließende Trocknung bzw. Calcination erhalten werden. Phosphorsäure kann aber auch mit Kieselgel zusammen in einem Sprühturm versprüht werden; danach schließt sich eine Trocknung und meist eine Calcination an. Phosphorsäure kann auch in einer Imprägniermühle auf das Trägermaterial aufgesprüht werden.

Die nachstehend beschriebenen Katalysatoren können wahlweise als 2- bis 4-mm-Stränge oder als Tabletten mit 3 bis 5 mm Durchmesser oder als Splitt mit Teilchengrößen von 0,1 bis 0,5 mm oder als Wirbelgut eingesetzt werden.

Herstellung der Katalysatoren

Katalysator 1

Der Borosilikatzeolith des Pentasil-Typs wird in einer hydrothermalen Synthese aus 650 g hochdispersem $SiO_2$, 373 g $H_3BO_3$, 8000 g einer wäßrigen 1,6-Diaminohexan-Lösung (50 Gew.% Amin) bei 165°C unter autogenem Druck in einem Rührautoklaven hergestellt. Nach Abfiltrieren und Auswaschen wird das kristalline Reaktionsprodukt bei 100°C/24 h getrocknet und bei 500°C/24 h calciniert. Dieser Borosilikatzeolith setzt sich zusammen aus 95,4 Gew.% $SiO_2$ und 3,31 Gew.% $B_2O_3$.

Mit diesem Material werden durch Verformen mit einem Verformungshilfsmittel 2-mm-Stränge hergestellt, die bei 110°C/16 h getrocknet und bei 500°C 16 h calciniert werden.

Katalysator 2

Die bei Katalysator 1 hergestellten Borosilikatzeolith-Stränge werden mit einer wäßrigen $Pd(NO_3)_2$-Lösung getränkt, bei 130°C im Drehrohr 1 h getrocknet und dann bei 540°C 1 h calciniert. Der Pd-Gehalt beträgt 2,30 Gew.%.

Katalysator 3

Ein Aluminosilikatzeolith vom Pentasil-Typ wird unter hydrothermalen Bedingungen bei autogenem Druck und 150°C aus 650 g hochdispersem $SiO_2$, 203 g $Al_2(SO_4)_3$ x 18 $H_2O$ in 11,3 kg einer wäßrigen 1,6-Diaminohexan-Lösung (42 Gew.% Amin) in einem Rührautoklaven hergestellt. Nach Abfiltrieren und Auswaschen wird das kristalline Reaktionsprodukt bei 110°C/24 h getrocknet und bei 500°C/24 h calciniert. Dieser Aluminosilikatzeolith enthält 92,6 Gew.% $SiO_2$ und 4,6 Gew.% $Al_2O_3$.

Mit diesem Material werden durch Verformen mit einem Verformungshilfsmittel 2-mm-Stränge hergestellt, die bei 110°C 16 h getrocknet und bei 500°C 16 h calciniert werden.

Katalysator 4

Ein Eisensilicatzeolith vom Pentasil-Typ wird unter hydrothermalen Bedingungen bei autogenem Druck und 165°C aus 273 g Natrium-Wasserglas (27,2 Gew.% $SiO_2$, 8,5 Gew.% $Na_2O$), 126 g 1,6-Diaminohexan, 551 g Wasser, 20,6 g 96 %iger Schwefelsäure und 31,1 g Eisen(III)-sulfat in einem Rührautoklaven hergestellt. Nach Abfiltrieren und Auswaschen wird das kristalline Reaktionsprodukt bei 110°C/24 h getrocknet und bei

500°C/24 h calciniert. Dieser Eisensilicatzeolith setzt sich aus 84,5 Gew.% $SiO_2$, 12,1 Gew.% $Fe_2O_3$ und 0.074 Gew.% Na zusammen. Mit diesem Material werden durch Verformen mit einem Verformungshilfsmittel 4-mm-Stränge hergestellt, die bei 110°C/16 h getrocknet und bei 500°C/24 h calciniert werden.

Die Stränge werden viermal mit 20 gew.-%iger $NH_4Cl$-Lösung (15 ml Lösung/g Formkörper) bei 80°C in einem Steigrohr ausgetauscht. Anschließend wird chloridfrei gewaschen. Die Stränge werden bei 110°C 10 h getrocknet und anschließend bei 500°C 5 h calciniert. Der Na-Gehalt beträgt nun 0.015 Gew.%.

Katalysator 5

Ein handelsüblicher Zeolith Y in der Na-Form wird einer 20 gew.%igen $NH_4NO_3$-Lösung (Gewichtsverhältnis 1:5) 2 h bei 80°C °C ausgetauscht.Danach wird nitratfrei gewaschen, bei 110°C getrocknet und 2 h bei 590°C im Tiefbett calciniert. Es folgen drei weitere Austauschvorgänge ohne Calcinieren. Abschließend wird nitratfrei gewaschen, an Luft getrocknet und bei 850°C 4 h im Tiefbett calciniert. Der Na-Gehalt beträgt 0,10 Gew.%. Der Zeolith wird nun in 2 n $HNO_3$ (Gewichtsverhältnis 1:6) 3 h bei 95°C gerührt. Nach dem Absaugen wird mit Wasser gewaschen und an der Luft getrocknet. Der Zeolith wird mit 0,3 n $HNO_3$ (Gewichtsverhältnis 1:6) 3 h bei 95°C gerührt, abgesaugt, nitratfrei gewaschen und an der Luft getrocknet. Er wird 4 h bei 530°C im Flachbett calciniert. Das molare $SiO_2/Al_2O_3$-Verhältnis beträgt 149.

Mit diesem Zeolith werden durch Verformen mit einem Verformungshilfsmittel 2-mm-Stränge hergestellt. Diese werden bei 110°C getrocknet und 16 h bei 500°C calciniert.

Katalysator 6

Ein handelsüblicher Zeolith Y in der Na-Form wird mit 20 gew.%iger $NH_4(SO_4)_2$-Lösung (Gewichtsverhältnis 1:15) bei 80°C ausgetauscht. Mit diesem Material werden durch Verformen mit einem Verformungshilfsmittel 2-mm-Stränge hergestellt, die bei 110°C getrocknet und anschließend bei 500°C 16 h calciniert werden. Der Na-Gehalt beträgt 0,14 Gew.%.

Katalysator 7

Katalysator 7 ist ein handelsübliches $Al_2O_3$ (D 10-10®)

Katalysator 8

Katalysator 8 ist ein handelsübliches $SiO_2$ (D 11-10®)

Katalysator 9

Ein Aluminosilikatzeolith vom ZSM-11-Typ wird unter hydrothermalen Bedingungen bei autogenem Druck und 160°C aus 10,9 kg Wasser, 6,3 kg Natrium-Wasserglas (8,0 Gew.% $Na_2O$, 26,5 Gew.% $SiO_2$), 180 g Aluminiumsulfat-18-hydrat, 600 g Schwefelsäure konz. und 600 g 1,8-Diaminooctan in einem Rührautoklaven hergestellt. Nach Abfiltrieren und Auswaschen wird das kristalline Reaktionsprodukt bei 110°C 24 h getrocknet und bei 500°C 24 h calciniert. Dieser Zeolith ist durch folgende Zusammensetzung charakterisiert: 71,6 Gew.% $SiO_2$, 1,7 Gew.% $Al_2O_3$, 0,24 Gew.% $Na_2O$.

Mit diesem Material werden durch Verformen mit einem Verformungshilfsmittel 2-mm-Stränge hergestellt, die bei 110°C/16 h getrocknet und bei 500°C/16 h calciniert werden.

Die Stränge werden viermal mit 20 gew.-%iger $NH_4Cl$-Lösung (15 ml Lösung/g Formkörper) bei 80°C in einem Steigrohr ausgetauscht. Anschließend wird chloridfrei gewaschen. Die Stränge werden bei 110°C 10 h getrocknet und anschließend bei 500°C 5 h calciniert. Der Na-Gehalt beträgt nun 0.015 Gew.%.

Katalsyator 10

Ein SAPO-11 wird synthetisiert, indem man 200 g Orthophosphorsäure (98 Gew.%) mit 417 g Aluminiumtriisopropylat und 60 g Kieselsol (30 Gew-% $SiO_2$) in 927 g Wasser homogen mischt; zu dieser Mischung werden 91,5 g Di-n-propylamin hinzugefügt. Danach wird bei 200°C 96 h unter autogenem Druck in einem Rühr-Autoklaven umgesetzt. Das abfiltrierte Siliciumaluminiumphosphat wird getrocknet bei 110°C und calciniert bei 550°C. Die Zusammensetzung des SAPO-11 ist 35,9 Gew.% $Al_2O_3$, 47,7 Gew.% $P_2O_5$ und 2,98 Gew.% $SiO_2$. Mit diesem Material werden durch Verformen mit einem Verformungshilfsmittel 2-mm-Stränge hergestellt, die bei 110°C/16 h getrocknet und bei 500°C/16 h calciniert werden.

Katalysator 11

$BPO_4$ wird hergestellt, indem man 49 g $H_3BO_3$ mit 117 g $H_3PO_4$ (75 %ig) in einem Kneter zusammengibt, überschüssiges Wasser abdampft und das Umsetzungsprodukt zu 3-mm-Strängen verformt. Diese Stränge werden bei 100°C getrocknet und bei 350°C calciniert. Der Katalysator enthält 8,77 Gew.% B und 28,3 Gew.% P.

Beispiele

Beispiele 1 bis 17

Gasphasenreaktionen zur Herstellung von Benzylmethylethern aus Benzaldehyddimethylacetalen

Die Reaktionen in der Gasphase wurden bei Normaldruck unter isothermen Bedingungen in einem Rohrreaktor (Wendel, 0,6 cm Innendurchmesser, 90 cm Länge) mehrere Stunden lang an einem Katalysatorfestbett durchgeführt. Dabei wurde die Katalysatormenge zwischen 1 und 10 g, entsprechend einer Belastung WHSV zwischen von 15 und 0.5/h variiert. Die Reaktionsprodukte wurden durch übliche Methoden abgetrennt und durch GC/MS, NMR oder Siedepunkt charakterisiert. Die quantitative Bestimmung der Reaktionsprodukte und der Ausgangsprodukte erfolgte gaschromatographisch oder durch Auswiegen der durch Destillation oder Extraktion erhaltenen Fraktionen.

Zur Synthese der Benzylmethylether im vorstehend beschriebenen Rohrreaktor werden bei Normaldruck mit einer Dosierpumpe 10 ml/h des jeweiligen Dimethylacetals unter gleichzeitigem Inertisieren mit einem Stickstoffstrom dem Reaktor kontinuierlich zugeführt und darin verdampft. Das mit 4 g Katalysator beschickte Festbett wird dabei je nach Fahrweise entweder aufsteigend oder absteigend von den gasförmig vorliegenden Edukten und Reaktionsprodukten durchströmt. Nach der Umsetzung am Katalysator bei 250°C wird der Reaktionsaustrag in einer Glasapparatur kondensiert und nach der in Stunden [h] angegebenen Laufzeit gaschromatisch analysiert. Die Umsätze und Selektivitäten sind in den Tabellen in Flächenprozenten angegeben.

Beispiele 1 bis 10

Herstellung von 2-Methoxybenzylmethylether aus 2-Methoxybenzaldehyddimethylacetal

| Beispiel | Zeit [h] | Katalysator | Umsatz | Selektivität | Ausbeute [%] |
|---|---|---|---|---|---|
| 1 | 2 | 1 | 36.0 | 53.9 | 19.4 |
| 2 | 6 | 2 | 16.2 | 78.8 | 12.8 |
| 3 | 2 | 3 | 93.3 | 42.3 | 39.5 |
| | 4 | | 66.6 | 65.0 | 43.3 |
| | 6 | | 54.9 | 73.8 | 40.5 |
| 4 | 2 | 4 | 80.1 | 43.0 | 34.5 |
| 5 | 2 | 5 | 29.4 | 54.6 | 16.1 |
| 6 | 2 | 6 | 24.9 | 32.8 | 8.2 |
| 7 | 2 | 7 | 20.5 | 55.4 | 11.4 |
| 8 | 2 | 8 | 61.1 | 52.5 | 32.1 |
| | 4 | | 31,7 | 67,2 | 21,3 |
| 9 | 2 | 9 | 29.8 | 54.1 | 16.1 |
| 10 | 2 | 11 | 40.5 | 17.0 | 6.9 |

Beispiele 11 bis 12

Herstellung von 4-Methoxybenzylmethylether aus 4-Methoxybenzaldehyddimethylacetal

| Beispiel | Zeit [h] | Katalysator | Umsatz | Selektivität | Ausbeute [%] |
|---|---|---|---|---|---|
| 11 | 2 | 1 | 79.5 | 43.4 | 34.5 |
| | 4 | | 52.3 | 51.1 | 26.7 |
| | 6 | | 43.9 | 50.3 | 22.1 |
| 12 | 2 | 3 | 69.8 | 49.6 | 34.7 |

Beispiele 13 bis 15

Herstellung von 4-Methylbenzylmethylether aus 4-Methylbenzaldehyddimethylacetal

| Beispiel | Zeit [h] | Katalysator | Umsatz | Selektivität | Ausbeute [%] |
|---|---|---|---|---|---|
| 13 | 2 | 1 | 67.4 | 59.1 | 39.8 |
| 14 | 2 | 3 | 99.6 | 34.7 | 34.6 |
| | 4 | | 91.6 | 51.7 | 47.4 |
| | 6 | | 70.0 | 55.2 | 38.7 |
| 15 | 2 | 10 | 54.6 | 39.0 | 21.1 |

Beispiele 16 bis 17

Herstellung von 4-t.-Butylbenzylmethylether aus 4-t-Butylbenzaldehyddimethylacetal

| Beispiel | Zeit [h] | Katalysator | Umsatz | Selektivität | Ausbeute [%] |
|---|---|---|---|---|---|
| 16 | 2 | 1 | 68.8 | 56.7 | 39.0 |
| 17 | 2 | 3 | 98.1 | 76.2 | 74.8 |
| | 4 | | 86.3 | 58.1 | 50.1 |

Beispiele 17

Herstellung von 2-Chlor-3-Cyclopentylbenzylmethylether aus 2-Chlor-3-Cyclopentylbenzaldehyddimethylacetal

| Beispiel | Zeit [h] | Katalysator | Umsatz | Selektivität | Ausbeute [%] |
|---|---|---|---|---|---|
| 17 | | 3 | 57,7 | 34,7 | 20 |

**Patentansprüche**

1. Verfahren zur Herstellung von Benzylmethylethern der allgemeinen Formel I

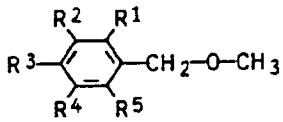

(I)

in denen $R^1$ bis $R^5$ Alkyl, Cycloalkyl, Alkylaryl, Aryl, Alkoxy, Wasserstoff, Hydroxy oder Halogen bedeutet, dadurch gekennzeichnet, daß man Benzaldehyddimethylacetale der allgemeinen Formel II

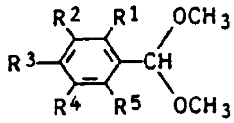

(II),

in denen $R^1$ bis $R^5$ die vorgenannte Bedeutung aufweisen, in der Gasphase an sauren Heterogenkatalysatoren bei Temperaturen von 50 bis 500°C und Drücken von 0,01 bis 50 bar umsetzt.

2. Verfahren nach Anspruch 1, dadurch gekennzeichnet, daß man als Heterogenkatalysatoren Zeolithe des Pentasil-Typs oder des Faujasit-Typs verwendet.

3. Verfahren nach Anspruch 1, dadurch gekennzeichnet, daß man als Heterogenkatalysator Aluminosilikatzeolithe, Borosilikatzeolithe, Eisensilikatzeolithe oder Galliumsilikatzeolithe verwendet.

4. Verfahren nach Anspruch 1, dadurch gekennzeichnet, daß man als Heterogenkatalysatoren Zeolithe verwendet, welche Kationen der Alkalimetalle, der Erdalkalimetalle, der übergangsmetalle oder der Seltenerdmetalle enthalten.

5. Verfahren nach Anspruch 1, dadurch gekennzeichnet, daß man als Heterogenkatalysatoren mit Säuren behandelte Zeolithe verwendet.

6. Verfahren nach Anspruch 1, dadurch gekennzeichnet, daß man als Heterogenkatalysatoren mit Phosphor dotierte Zeolithe verwendet.

7. Verfahren nach Anspruch 1, dadurch gekennzeichnet, daß man als Heterogenkatalysatoren Phosphate der Elemente B, Al, Ce, Fe, Sr, Zr oder deren Gemische verwendet.

8. Verfahren nach Anspruch 1, dadurch gekennzeichnet, daß man als Heterogenkatalysatoren hydrothermal hergestellte Aluminophosphate oder Siliciumaluminophosphate verwendet, die gegebenenfalls Metallionen der Elemente Co, Fe, Mg, Mn, Zn, B, Be, Ga, As oder Ti enthalten.

9. Verfahren nach Anspruch 1, dadurch gekennzeichnet, daß man als Heterogenkatalysatoren $SiO_2$, $Al_2O_3$ oder amorphe Aluminosilikate verwendet, die gegebenfalls mit Phosphorsäure oder Borsäure imprägniert sind.

## Claims

1. A process for the preparation of benzyl methyl ethers of the formula I

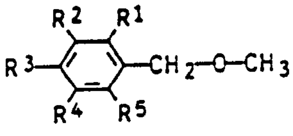

(I)

where $R^1$ to $R^5$ are alkyl, cycloalkyl, alkylaryl, aryl, alkoxy, hydrogen, hydroxyl or halogen, which comprises reacting benzaldehyde dimethyl acetals of the formula II

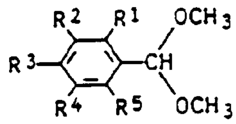

(II),

where $R^1$ to $R^5$ are as defined above, in the gas phase on acidic heterogeneous catalysts at from 50 to 500°C and at from 0.01 to 50 bar.

2. A process as claimed in claim 1, wherein the heterogeneous catalysts used are zeolites of the pentasil type or of the faujasite type.

3. A process as claimed in claim 1, wherein the heterogeneous catalysts used are aluminosilicate zeolites, borosilicate zeolites iron silicate zeolites or gallium silicate zeolites.

4. A process as claimed in claim 1, wherein the heterogeneous catalysts used are zeolites containing cations of the alkali metals, alkaline earth metals, transition metals or rare-earth metals.

5. A process as claimed in claim 1, wherein the heterogeneous catalysts used are acid-treated zeolites.

6. A process as claimed in claim 1, wherein the heterogeneous catalysts used are phosphorus-doped zeolites.

7. A process as claimed in claim 1, wherein the heterogeneous catalysts used are phosphates of the elements B, Al, Ce, Fe, Sr and Zr or mixtures thereof.

8. A process as claimed in claim 1, wherein the heterogeneous catalysts used are hydrothermally prepared aluminophosphates or silicon aluminophosphates, if desired containing metal ions of the elements Co, Fe, Mg, Mn, Zn, B, Be, Ga, As or Ti.

9. A process as claimed in claim 1, wherein the heterogeneous catalysts used are $SiO_2$, $Al_2O_3$ or amorphous alumino silicates, if desired impregnated with phosphoric acid or boric acid.

## Revendications

1. Procédé de préparation d'oxydes de méthyle et de benzyle de formule générale I

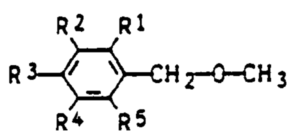

(I)

dans laquelle $R^1$ à $R^5$ représentent chacun un reste alkyle, cycloalkyle, alkylaryle, aryle, alcoxy, un atome d'hydrogène, un groupement hydroxy ou un atome d'halogène, caractérisé en ce qu'on fait réagir des benzaldéhyde-diméthylacétals de formule générale II

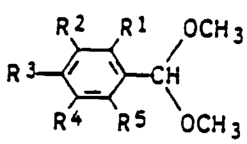

(II),

dans laquelle $R^1$ à $R^5$ ont les significations sus-indiquées, en phase gazeuse, en présence de catalyseurs hétérogènes acides, à des températures de 50 à 500°C et sous des pressions de 0,01 à 50 bar.

2. Procédé selon la revendication 1, caractérisé en ce qu'on utilise, comme catalyseurs hétérogènes, des zéolithes du type pentasil ou du type faujasite.

3. Procédé selon la revendication 1, caractérisé en ce qu'on utilise, comme catalyseurs hétérogènes, des zéolithes d'aluminosilicate, des zéolithes de borosilicate, des zéolithes de ferrosilicate ou des zéolithes de silicate de gallium.

4. Procédé selon la revendication 1, caractérisé en ce qu'on utilise, comme catalyseurs hétérogènes, des zéolithes qui contiennent des cations des étaux alcalins, des métaux alcalino-terreux, des métaux de transition ou des étaux des terres rares.

5. Procédé selon la revendication 1, caractérisé en ce qu'on utilise, comme catalyseurs hétérogènes, des zéolithes traitées par des acides.

6. Procédé selon la revendication 1, caractérisé en ce qu'on utilise, comme catalyseurs hétérogènes, des zéolithes dopées avec du phosphore.

7. Procédé selon la revendication 1, caractérisé en ce qu'on utilise, comme catalyseurs hétérogènes, des phosphates des éléments B, Al, Ce, Fe, Sr, Zr ou leurs mélanges.

8. Procédé selon la revendication 1, caractérisé en ce qu'on utilise, comme catalyseurs hétérogènes, des phosphates d'aluminium ou des phosphates d'aluminium et de silicium qui ont été préparés par synthèse hydrothermale et qui contiennent éventuellement des ions métalliques des éléments Co, Fe, Mg, Mn, Zn, B, Be, Ga, As ou Ti.

9. Procédé selon la revendication 1, caractérisé en ce qu'on utilise, comme catalyseurs hétérogènes, $SiO_2$, $Al_2O_3$ ou des aluminosilicates amorphes qui sont éventuellement imprégnés d'acide phosphorique ou d'acide borique.